(19) **European Patent Office** (11) Veröffentlichungsnummer: **0 066 249**

Office européen des brevets **A2**

(12) **EUROPÄISCHE PATENTANMELDUNG**

(21) Anmeldenummer: **82104582.0** (51) Int. Cl.³: **C 12 N 15/00**

(22) Anmeldetag: **26.05.82**

---

(30) Priorität: **02.06.81 DE 3121815**

(43) Veröffentlichungstag der Anmeldung:
**08.12.82 Patentblatt 82/49**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt/Main 80(DE)**

(72) Erfinder: **Esser, Karl, Prof.Dr.**
**Am Spik 23a**
**D-4630 Bochum(DE)**

(72) Erfinder: **Stahl, Ulf, Dr.**
**Biermannsweg 22**
**D-4630 Bochum(DE)**

(72) Erfinder: **Tudzynski, Paul, Dr.**
**Meerstrasse 123**
**D-4390 Gladbeck(DE)**

(72) Erfinder: **Kück, Ulrich**
**Haspelstrick 20**
**D-4630 Bochum(DE)**

---

(54) **Hybridvektor, Verfahren zur Verbesserung der Amplifikation und Expression von Hybridvektoren durch die Verwendung von mitochondrialer DNA.**

**EP 0 066 249 A2**

Croydon Printing Company Ltd.

0066249

HOECHST AKTIENGESELLSCHAFT HOE 81/F 134      Dr.KL/cr

## Hybridvektor, Verfahren zur Verbesserung der Amplifikation und Expression von Hybridvektoren durch die Verwendung von mitochondrialer DNA

Es ist bereits seit den ersten Beobachtungen von Avery et al. (J.exp.Med. 79, 137-158 (1944)) bekannt, daß genetische Informationen von einem Bakterium auf ein anderes übertragen werden können. Hiermit war die Grundlage für in den letzten Jahren entwickelte Techniken geschaffen worden, mit denen gezielt genetische Informationen von einem Organismus auf einen anderen übertragen werden können. Diese auch als "genetische Manipulationen" bezeichneten Arbeitsweisen sind z.B. in dem Lehrbuch von Klingmüller "Genmanipulation und Gentherapie" Springer-Verlag, 1976, im einzelnen beschrieben.

Der entscheidende Schritt bei allen gentechnologischen Arbeiten besteht darin, DNA mit Hilfe eines Vektors in einen neuen Wirtsorganismus einzuführen. Als solche Vektoren werden bisher meist bakterielle Plasmide oder doppelsträngige DNA aus Bakteriophagen, d.h. prokaryontische DNA verwendet. Eine wesentliche Voraussetzung für den Einsatz solcher Vektoren war die Entdeckung der Restriktionsenzyme von Arber und Linn (Ann.Rev.Biochem. 38, 467-500 (1969)). Hierbei handelt es sich um Endonukleasen, die die genannten Vektoren an spezifischen Stellen aufschneiden, ohne ihre Molekularstruktur im übrigen zu zerstören. Nach Vermischung von ebenfalls mit Endonukleasen behandelter, einem anderen Organismus entnommener DNA und Zugabe von Enzymen, die den aufgeschnittenen Vektor mit den DNA-Bruchstücken verbinden, den Ligasen, bildet sich ein neuer, zum Teil andere DNA-Bruchstücke enthaltender Vektor, ein sogenannter Hybridvektor. Derartige Hybridvektoren können dann in einen Wirtsorganismus übertragen werden und sich dort vermehren (amplifizieren), und zwar deswegen, weil die prokaryontische Vektor-DNA einen Replikationspunkt (origin of replication) besitzt. In günstigen Fällen ist diese Vermehrung im Wirtsorganismus (Klonierung) auch mit einer Expression der auf der eingeführten Fremd-DNA liegenden Gene verbunden.

Bisher wurden als Wirtsorganismen für die Aufnahme von Hybridvektoren, deren Amplifikation und zur Expression der Fremd-DNA fast ausschließlich Bakterien verwendet, und zwar verschiedene Stämme von Escherichia coli und von Bacillus subtilis. So ist z.B. bekannt, daß die genetische Information, die für die Bildung des Proinsulins notwendig ist und aus tierischen Zellen stammt, mit Hilfe eines Vektors in E. coli übertragen werden, und sich dort exprimieren kann (Esser und Stahl: "Hybridization", in "Handbook of Biotechnology", Vol. I, (1981)). Allerdings haben sich bei dieser Methode folgende Schwierigkeiten ergeben, die auch für die Praxis Bedeutung haben:

a) Es hat sich gezeigt, daß die DNA aus Prokaryonten und Eukaryonten bezüglich ihres Informationsgehaltes unterschiedliche Strukturen haben. Während bei Prokaryonten im Verlauf der genetischen Expression die gesamte DNA in Boten-RNA umgeschrieben wird (Transcription), ist dies bei Eukaryonten nicht der Fall. Die doppelsträngige DNA hat die genetische Information nicht kontinuierlich gespeichert, sondern besteht aus in-formationshaltigen Abschnitten (Exons) und informations-freien Abschnitten (Introns). Bei der Übertragung dieser Exons und Introns in einen Prokaryonten kann es deshalb zu Störungen seiner Proteinsynthese kommen.

b) Bakterien besitzen im Gegensatz zu den Eukaryonten nicht die 80 S-Ribosomen, die bei höheren Organismen eine Rolle bei der Proteinsynthese spielen, sondern nur die 70 S-Ribosomen, die den mitochondrialen Ribosomen der höheren Organismen entsprechen. Auch dies könnte eine Erklärung dafür sein, daß oft die Fremd-DNA in Prokaryonten·zwar amplifiziert, trotzdem aber nur eine geringe
Expression der Genprodukte festgestellt wird.

c) Außerdem wurde auch beobachtet, daß trotz Amplifikation
und Expression die Genprodukte der Eukaryonten-DNA
gelegentlich in Prokaryonten sekundär zerstört wurden.

d) Weiterhin muß man berücksichtigen, daß sich z.B. bei
Bacillus subtilis nach erfolgreicher Übertragung von
Eukaryonten-DNA in manchen Fällen eine Instabilität
der Transformation gezeigt hat, die dazu führte, daß die
übertragene genetische Information schon nach wenigen
Generationen in der Bakterienpopulation·verloren geht.

Diese Schwierigkeiten sind zusammengefaßt dargestellt
von Macleod /"Nature", 285, 136 (1980) 7.

Es stellte sich deshalb die Aufgabe, diese Nachteile
zu überwinden, um eine breitere Anwendung der Genmanupulation eukaryontischer Systeme zu ermöglichen.
Dies erfordert Vektoren, die sich in eukaryontischen
Zellen gut amplifizieren und auch exprimieren. Die
bisher zur Überwindung derartiger Schwierigkeiten entwickelten Systeme haben die in sie gesetzten Hoffnungen
nicht erfüllen können.

So gibt es zwar bei Saccharomyces cerevisiae (Bäckerhefe) ein eukaryontisches Plasmid, die sogenannte
2 μ-DNA, die sich für Hefe selbst als Vektor verwenden
läßt (Hollenberg, "Progress in Botany", 42, 171-185
(1980)). Dieses System konnte jedoch nach Übertragung
von genetischem Material aus tierischen Zellen, das
für das ß-Globin kodiert, bei der Transcription nicht
exakt zwischen Introns und Exons unterscheiden, d.h.,
die tierische DNA wurde nicht genau "gespleist".
/ Beggs et al. "Nature " 283, 835-840 (1980) 7. Außerdem haben derartige Hybridvektoren sich auch in Hefezellen nur schlecht vermehren lassen.

- 4 -

Auch der Versuch, tierische Zellen als Wirtsorganismen zu verwenden und dabei Viren als Vektoren einzusetzen, hat sich bisher schon wegen des großen damit verbundenen Aufwandes nicht durchsetzen können.

Es wurde nun gefunden, daß die vorstehend genannten Schwierigkeiten durch einen Hybridvektor überwunden werden können, der aus einem einen Replikationspunkt aufweisenden Segment einer mitochondrialen DNA und ggf. einem aus einem bakteriellen Plasmid gebildeten Segment aufgebaut ist.

Mitochondriale DNA ist als doppelsträngige, eukaryontische DNA gut geeignet, die Aufgabe eines Vektors zu übernehmen, da sie nicht in den Chromosomenverband integriert ist und in einer relativ hohen Kopienzahl in allen Zellen vorhanden ist. Sie ist außerdem als zirkuläre DNA leicht von anderer DNA abzutrennen und vor allem ist sie, da sie einen Replikationspunkt aufweist, in der Lage, sich selbst zu amplifizieren.

In ihrer Struktur nimmt die mitochondriale DNA eine Stellung zwischen chromosomaler und prokaryontischer DNA ein. Sie weist nämlich sowohl Gene mit Introns als auch Gene ohne Introns auf. /¯Michaelis et al., "Progress in Botany" 42, 227-233 (1980)_7. Deshalb ist mitochondriale DNA geeignet, als Vektor sowohl für ein Eukaryonten- als auch für ein Prokaryonten-System verwendet zu werden.

Die mitochondriale DNA weist je nach Organismus eine Länge von 5 bis etwa 30 µm auf. Deshalb ist es aus technischen Gründen oft nicht möglich, das gesamte Ringmolekül als Vektor zu verwenden. Man muß daher Segmente aus der mitochondrialen DNA herausschneiden, die einen Replikationspunkt enthalten. Soweit die mitochondriale DNA bereits mit Hilfe von Restriktionsenzymen kartiert ist und damit auch die Lage des Replikationspunktes bekannt ist, können mit Hilfe von Endonukleasen ohne weiteres diejenigen Segmente der mitochondrialen DNA herausgeschnitten werden, die in der Lage sind, sich unter geeigneten Bedingungen zu amplifizieren. Ist die mitochondriale DNA allerdings noch nicht kartiert, dann muß zunächst ein geeignetes, einen Replikationspunkt tragendes Segment der mitochondrialen DNA ermittelt werden.

In besonders einfacher Weise ist nun ein derartiger Hybridvektor dann zugänglich, wenn man als mitochondriale DNA die in dem Hyphenpilz Podospora gefundene und bereits früher ausführlich beschriebene pl-DNA zur Herstellung eines mitochondrialen Vektors verwendet. Bei dieser pl- DNA handelt es sich um eine ringförmige, kovalent geschlossene DNA, die regelmäßig in alternden Myzelien dieses Pilzes angetroffen wird und überraschend viele Ähnlich- keiten mit den üblicherweise für gentechnologische Arbeiten verwendeten Bakterienplasmiden zeigt. Insbesondere ist in diesem Zusammenhang auf das Molekulargewicht von 2,4 kb, die Konturlänge von 0,75 µm und die Schwimmdichte von 1,699 g/ $cm^3$ der pl-DNA hinzuweisen. In jungen Podospora-Myzelien ist die pl-DNA ein integraler Bestandteil der mitochondrialen-DNA. Wenn sie freigesetzt wird, sterben die Podosporazellen ab. Freie pl-DNA hat einen eigenen Replikationspunkt, kann sich also selbst vermehren und ist daher auch in der Lage, nach Übertragung in andere Zellen über Hyphen- oder Protoplastenfusion dort Alterungserscheinungen auszulösen.

Aus einem derartigen pl-DNA-Molekül und einem bakteriellen Plasmid wie pBr 322 läßt sich nach an sich bekannten Methoden ein Hybridvektor aufbauen. Dieser Hybridvektor kann sowohl in Escherichia coli als auch in Podospora cloniert werden. Sowohl im Bakterium als auch im Pilz ist eine Expression der den beiden Segmenten des Hybrid- vektors entsprechenden Genprodukte feststellbar. Sowohl in Escherichia coli als auch in Podospora läßt sich nach Einführung des Hybridvektors eine Expression des Gens für Ampicillinresistenz infolge der Bildung einer
ß-Lactamase durch das auf dem bakteriellen Segment des
Hybridvektors liegende Resistenzgen für Ampicillin nachweisen.      Ferner wird im Pilz das Auftreten von
Alterungserscheinungen beobachtet, die auf das pl-DNA-
Segment des Hybridvektors zurückzuführen sind.

Es liegt auf der Hand, daß die Expression · von Alterungserscheinungen die gentechnologischen Arbeiten mit
Podospora-Arten erheblich behindert. Deshalb hat es sich
als vorteilhaft erwiesen, für den Aufbau von Hybridvektoren Podospora-Stämme zu verwenden, die eine Expression
der Seneszenz  nicht mehr zeigen. Derartige Mutanten
sind bei Podospora bekannt.

Es steht somit ein Verfahren zur Verbesserung der
Amplifikation und Expression von Hybdridvektoren zur
Verfügung, die Segmente von prokaryontischer und
eukaryontischer DNA enthalten, bei dem man in den Hybridvektor als eukaryontische DNA mitochondriale DNA einbaut
und diesen Hybridvektor in an sich bekannter Weise zur
Transformation einer prokaryontischen oder eurkaryontischen
Wirtszelle einsetzt. Ein derartiger Vektor kann sowohl
zur Übertragung prokaryontischer als auch zur Übertragung
eukaryontischer DNA auf eine geeignete Wirtszelle eingesetzt werden. Damit wird ein Weg gezeigt, wie Hindernisse,
die sich einer Anwendung gentechnologischer Methoden in der
Praxis bisher in den Weg gestellt haben, überwunden werden
können.

## 1. Isolation von pl-DNA aus Podospora anserina

Der Ausgangsstamm für die Gewinnung von pl-DNA ist der Wildstamm $s_1$ von Podospora anserina, wie er von Esser im "Handbook of Genetics", Vol. I, S. 531-551 (1974) beschrieben ist. Präseneszentes Myzel dieses Stammes wird etwa 3 Wochen lang auf einem Komplettmedium, wie es in der vorstehend genannten Veröffentlichung von Esser beschrieben ist, im Fernbachkolben bei 26°C angezogen. Hieraus wird die pl-DNA isoliert und gereinigt nach der von Stahl et al. in Molec.gen.Genet. 178,639 -646 (1980) angegebenen Arbeitsweise.

Die nach diesem Verfahren isolierte pl-DNA (Schwimmdichte in CsCl : 1,699 $g/cm^3$) ist hinsichtlich des Molekulargewichtes sehr heterogen. Sie besteht nämlich aus einer Reihe oligomerer, ringförmiger Moleküle, die jedoch alle die gleiche Grundeinheit mit einer Konturlänge von 0,75 μm zeigen. Es konnte beobachtet werden, daß bis zu 13 Grundeinheiten der genannten Konturlänge zu einem einzigen Molekül zusammentreten können.

Durch Verdauung mit bestimmten Restriktionsendonucleasen, die pro Grundeinheit nur eine einzige Schnittstelle haben wie Sal I (=Restriktionsenzym aus Streptomyces albus),
      Kpn I (= Restriktionsenzym aus Klebsiella pneumoniae) und Bgl II (= Restriktionsenzym aus Bacillus globigii) läßt sich die gesamte DNA derartiger Moleküle in DNA-Segmente der einheitlichen Grundgröße einer Konturlänge von 0,75 μm überführen.

## Herstellung eines Hybridvektors

Die Herstellung eines zur Clonierung in E.coli und Podospora geeigneten Vektors durch Verbindung des E.coli Vektors pBr 322 mit der isolierten pl-DNA ist bereits beschrieben worden von Stahl et al. in "Molec.gen.Genet".

178, 639-646 (1980).

Die Herstellung eines derartigen Hybridvektors aus dem E.coli Vektor pBr 322 und einem Restriktionsfragment (Sal 4) der mitochondrialen DNA, das einen Replikationspunkt trägt und zum Teil mit der pl-DNA homolog ist, ist bisher noch nicht bekannt. Hierfür ist folgende Arbeitsweise geeignet:

Es ist bekannt, daß die pl-DNA deswegen zur Konstruktion eines Vektors benutzt werden kann, weil sie einen Replikationspunkt trägt. Dementsprechend kann der Aufbau eines Hybridvektors aus der intakten (juvenilen) mitochondrialen DNA von Podospora nur dann zu einem Erfolg führen, wenn das dieser DNA entnommene Fragment ebenfalls einen Replikationspunkt enthält. Ein derartiges Fragment der juvenilen mitochondrialen DNA läßt sich durch die Behandlung mit dem Restriktionsenzym Sal I erhalten.

Es wurden deshalb das Plasmid pBr 322 und gereinigte, juvenile, mitochondriale DNA von Podospora anserina mit dem Restriktionsenzym Sal I geschnitten und die entstandenen Bruchstücke in der gleichen Weise ligiert, wie es in der vorstehend genannten Literaturstelle von Stahl beschrieben ist. Der so gebildete Hybridvektor wird in E.coli-Transformanden selektioniert, die Ampicillin-Resistenz aufweisen.Deren DNA wird isoliert und mittels Restriktionsanalyse charakterisiert. Dadurch werden diejenigen Hybridplasmide ausgewählt, die das zu übertragende DNA-Fragment, im vorliegenden Fall das Sal 4 -Fragment, tatsächlich aufgenommen haben.

Hybridvektoren, die dieses Fragment enthalten, zeigen eine ebenso gute Transformationsrate und Replikation in Podospora wie die schon früher hergestellten Hybridvektoren aus pBr 322 und der pl-DNA.

Das vorliegende Verfahren zur Herstellung eines Hybridvektors ist generell anwendbar auf beliebige mitochrondriale DNA, sofern deren Replikationspunkt bekannt
ist.

3. Transformation mit Hybridvektoren in Podospora

a) Rezipientenstämme

Das nachstehend beschriebene Verfahren kann im Prinzip mit
einem Wildstamm von Podospora anserina durchgeführt
werden; dann läßt sich die Expression des eingeführten
Hybridplasmids durch die Auslösung von Seneszenzsymptomen
nachweisen. Dies ist allerdings für die praktische
Anwendung ungeeignet, weil entsprechend transformierte
Stämme schnell irreversibel absterben.

Durch Verwendung von langlebigen Mutantenstämmen von
Podospora kann dieses Problem gelöst werden. Bisher wurden
hierfür die folgenden beiden Stämme eingesetzt:

- Die Podospora-Doppelmutante gr viv (grisea/vivax),
  die morphologisch beschrieben ist bei Tudzynski und
  Esser in Molec.gen.Genet. 173, 71-84, (1979) und
  hinterlegt ist als DSM 2099, zeigt keine spontanen
  Alterungserscheinungen und enthält keine freie pl-
  DNA. Sie läßt sich aber transformieren mit pl-DNA
  und Hybridvektoren und zeigt dann Seneszenzsymptome
  (Tudzynski et al. in Current Genet. 2,181-184, (1980)).
  Diese Symptome sind hier aber nicht so ausgeprägt
  wie beim Wildstamm und teilweise durch Kältebehandlung
  reversibel.

- Die Podospora-Doppelmutante i viv (incoloris vivax),
  die morphologisch beschrieben ist von Esser und
  Keller in Molec.gen.Genet. 144, 107-110 (1976) und
  hinterlegt ist als DSM 2098, zeigt keine spontane

- 10 -

und keine induzierte Seneszenz. Da sich p1-DNA aber in ihr replizieren kann, ist sie hervorragend als Rezipientenstamm geeignet. Natürlich läßt sich hier die Seneszenzauslösung nicht als Selektionsmarke für Transformanden verwenden.

b) Transformation

Der Rezipientenstamm wird in Fernbachkolben auf einem Komplettmedium 4 bis 5 Tage bei 26°C angezogen. Das geerntete Mycel wird in einer Protoplastenpufferlösung aufgenommen, dann etwa 20 Sekunden homogenisiert und schließlich 3 Stunden mit einem lytischen Enzymgemisch aus Trichoderma harzianum zur Herstellung von Protoplasten inkubiert.

Dann wurden die Protoplasten zweimal mit der Protoplastenpufferlösung (0,5 Mol Sucrose gelöst in einer 0,1 molaren Lösung von Tris-maleat, welche mit NaOH auf einen pH 7 eingestellt ist) gewaschen. Anschließend wurden die Protoplasten in dieser Pufferlösung, der noch 10 mmol Calciumchlorid zugegeben wurden, bis zu einer Endkonzentration von $5 \times 10^6$ bis $5 \times 10^7$ resuspendiert. Hierzu wurde die Hybrid-DNA, gelöst in TES-Puffer (0,05 M Tris, 0,005 Mol EDTA, 0,05 Mol Natriumchlorid, pH 8) bis zu einer Konzentration von 5 - 10 µg/ml (Wildstamm) oder 40 µg/ml (Doppelmutante gr viv) gegeben. In Vergleichsversuchen wurde der TES-Puffer ohne irgendeine Hybrid-DNA angewendet. Die Mischung blieb 15 Minuten bei 26°C stehen. Danach wurde die Suspension im Verhältnis 1:10 mit PEG-Puffer verdünnt (20 % Polyäthylenglycol 4000, 10 mMol Calciumchlorid, 10 mMol Tris-HCl, pH 7,5) und 1 Stunde bei 26°C inkubiert. Anschließend wurden die Protoplasten auf ein Regenerationsmedium aufgetragen.

Das Vorhandensein eines Gens für eine ß-Lactamase auf dem prokaryontischen Segment des Hybridvektors konnte durch folgendes Prüfverfahren nachgewiesen werden:

Dem Regenerationsmedium für Protoplasten wird Sorbose zugesetzt (15 g/l) wodurch die entstehenden Kolonien klein und abgegrenzt bleiben. Dann wird ein ß-Lactamase-Nachweisreagenz aufgesprüht oder in einer dünnen Schicht Weichagar aufgetragen, z.B. Padac (Schindler und Huber in "Enzyme Inhibitors", 169 - 176 (1980)) oder Nitrocefin /⁻O'Callaghan et al. in "Antimic. Agent Chemoth 1.", 283-288 (1972) ·7. Diese Reagenzien zeigen einen spezifischen Farbumschlag bei Anwesenheit von ß-Lactamasen, Nitrocefin z.B. von gelb nach rot. Auf diese Weise lassen sich transformierte Kolonien identifizieren, da Podospora (wie alle Eukaryonten) keine eigene ß-Lactamase synthetisiert. Eine gesteigerte Empfindlichkeit des Nachweisverfahrens wird erreicht, wenn die Kolonien einige Stunden mit einem Filter bedeckt werden, der mit dem obengenannten lytischen Enzymgemisch getränkt ist. Der ß-Lactamasetest kann dann direkt auf dem Filter durchgeführt werden.

Die Selektion von Transformanden kann auch durch zusätzlich in den Vektor eingebaute eukaryotische Gen-Marken erfolgen, z.B. durch Komplementation einer Auxotrophiemutation im Rezipientenstamm durch ein entsprechendes Wildgen auf dem Vektor.

Die so identifizierten Transformanden werden vereinzelt und angezogen. Sie müssen das Hybridplasmid enthalten, da seine Amplifikation und Expression durch den ß-Lactamasetest impliziert wird. Aus solchen Transformanden wird DNA isoliert, die in Dichte und Restriktionsmuster dem eingesetzten Hybridplasmid entspricht und die bei Rücktransformation in E.coli zur Bildung von Ampicillin-resistenten Kolonien führt. Aus diesen Kolonien gewonnene Plasmid-DNA enthält Sequenzen, die der ursprünglich eingesetzten Podospora-DNA homolog sind.

Die beschriebenen Hybridplasmide sind also sowohl zur Amplifikation und Expression in Podospora als auch in E.coli geeignet.

## 4. Herstellung eines Hybridvektors mit einem Replikationsursprung der mt-DNS von Acremonium chrysogenum

Um von einem beliebigen Eukaryonten ein Restriktionsfragment zu erhalten, das einen Replikationsursprung enthält, kann man neben dem direkten Transformationstest im endgültigen Empfängerorganismus (wie für Podospora beschrieben) auch eine Vorselektion in der Bäckerhefe Saccharomyces cerevisiae durchführen.

Dieser eukaryotische Mikroorganismus ist experimentell sehr leicht zu handhaben, vor allem kann man auf ein gut etabliertes Transformationssystem zurückgreifen. Dieses Hefetransformationssystem ist bereits ausführlich von Hinnen et al. beschrieben worden (Proc. Nat. Acad. Sci. US 75, 1929-1933, 1978). Es umfaßt den Leucin-auxotrophen Empfängerstamm AH-22 und Vektoren mit dem Hefe-Leucin-2-Gen aus dem Wildstamm von S. cerevisiae.

Bei der erfindungsgemäßen Anwendung benutzt man Aus- gangsvektoren, die keinen eukaryotischen Replikations- ursprung enthalten. Diese können sich in Hefe nur vermehren, wenn man in sie ein Restriktionsfragment integriert, das einen solchen enthält. Bei dem Hyphenpilz Acremonium chrysogenum wurde dieses Verfahren zur Isolation eines Replikationsursprungs der mt-DNS angewandt, und zwar auf folgende Weise:

Die mitochondriale DNS von Acremonium chrysogenum Gams (syn. Cephalosporium acremonium Corda) (Gams: Cephalosporiumartige Schimmelpilze, Fischer, Stuttgart 1971) hat eine Größe von 26,7 kb und wird durch das Restriktionsenzym PstI in drei Fragmente mit 24,5; 1,85; 0,53 kb zerlegt. Die erhaltenen Fragmente wurden mit dem ebenfalls durch PstI geschnittenen Vektor pDAM1

- 13 -

(Beach et al.: Nature 284, 185-187, 1980) wie oben beschrieben ligiert. Dieser Vektor besteht aus dem bakteriellen Plasmid pBR325 (Bolivar: Gene 4, 121-136, 1978) und dem Hefe-Leucin-2-Gen (s. oben). Er kann sich in Hefe nicht vermehren, da er keinen eukaryotischen Replikationsursprung enthält. Transformationsexperimente am Hefestamm AH-22 mit Hybridvektoren aus pDAM1 und Fragmenten der mt-DNS sind nur dann erfolgreich, wenn das integrierte Fragment einen eukaryotischen Replikationsursprung enthält. Im vorliegenden Fall konnte gezeigt werden, daß dies bei A. chrysogenum auf das PstI/2-Fragment zutrifft. Hybridvektoren mit diesem Fragment transformieren nämlich S. cerevisiae mit hoher Effizienz ( > 200 Transformanden pro µg DNS). Aus den Transformanden läßt sich freie Plasmid-DNS isolieren, die mit der eingesetzten DNS identisch ist. Das PstI/2-Fragment enthält demnach offensichtlich einen eukaryotischen Replikationsursprung.

Dieses Verfahren läßt sich im Prinzip mit jeder beliebigen mitochondrialen DNS durchführen.

PATENTANSPRÜCHE:

1. Hybridvektor, dadurch gekennzeichnet, daß er ein einen Replikationspunkt aufweisendes Segment einer mitochondrialen DNA enthält.

2. Hybridvektor nach Anspruch 1, dadurch gekennzeichnet, daß er ein aus einem prokaryontischen Plasmid gebildetes Segment enthält.

3. Hybridvektor nach Anspruch 2, dadurch gekennzeichnet, daß das Segment des prokaryontischen Plasmids ein Segment eines bakteriellen Plasmids ist.

4. Hybridvektor nach Anspruch 1 bis 3, dadurch gekennzeichnet, daß er ein aus der mitochondrialen DNA von Podospora- oder Acremoniumarten durch Einwirkung eines Restriktionsenzyms gewonnenes, einen Replikationspunkt aufweisendes Bruchstück enthält.

5. Verfahren zur Verbesserung der Amplifikation und Expression von Hybridvektoren, dadurch gekennzeichnet, daß man in den Hybridvektor ein einen Replikationspunkt aufweisendes Segment einer mitochondrialen DNA einbaut und diesen Hybridvektor in an sich bekannter Weise zur Transformation einer Wirtszelle einsetzt.

6. Verwendung von mitochondrialer DNA als Vektor zur Übertragung von DNA auf eine geeignete Wirtszelle.

7. Verwendung von mitochondrialer DNA oder eines Segmentes mitochondrialer DNA aus Acremonium- oder Podosporaarten als Vektor gemäß Anspruch 6.

0066249

Patentansprüche für Österreich:

1. Verfahren zur Verbesserung der Amplifikation und
   Expression von Hybridvektoren, dadurch gekennzeichnet,
   daß man in den Hybridvektor ein einen Replikationspunkt aufweisendes Segment einer mitochondrialen DNA
   einbaut und diesen Hybridvektor in an sich bekannter
   Weise zur Transformation einer Wirtszelle einsetzt.

2. Verfahren nach Anspruch 1, dadurch gekennzeichnet, daß
   der Hybridvektor ein aus einem prokaryontischen Plasmid
   gebildetes Segment enthält.

3. Verfahren nach Anspruch 2, dadurch gekennzeichnet,
   daß der Hybridvektor ein aus einem bakteriellen
   Plasmid gebildetes Segment enthält.

4. Verfahren nach Anspruch 1 bis 3, dadurch gekennzeichnet, daß der Hybridvektor mitochondriale DNA
   aus Podospora- oder Acremoniumarten enthält.

5. Verfahren nach Anspruch 1 bis 4, dadurch gekennzeichnet, daß der Hybridvektor ein aus der mitochondrialen
   DNA aus Acremonium- oder Podosporaarten durch Einwirkung eines Restriktionsenzyms gewonnenes, einen
   Replikationspunkt aufweisendes Bruchstück enthält.